(19) Europäisches Patentamt
European Patent Office
Office européen des brevets

(11) Veröffentlichungsnummer: **0 096 280**
**A2**

(12) # EUROPÄISCHE PATENTANMELDUNG

(21) Anmeldenummer: 83105089.3

(22) Anmeldetag: 24.05.83

(51) Int. Cl.³: C 07 D 249/08
C 07 D 249/18, C 07 D 235/06
C 07 D 233/54, C 07 D 233/92
C 07 D 231/12, A 01 N 47/44

(30) Priorität: 04.06.82 DE 3221139
29.01.83 DE 3303064

(43) Veröffentlichungstag der Anmeldung:
21.12.83 Patentblatt 83/51

(84) Benannte Vertragsstaaten:
AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Oeckl, Siegfried, Dr.
Auf der Hoehe 74
D-5060 Bergisch-Gladbach 1(DE)

(72) Erfinder: Schmitt, Hans-Georg, Dr.
Gustav-Freytag-Strasse 2
D-5090 Leverkusen 1(DE)

(72) Erfinder: Brandes, Wilhelm, Dr.
Eichendorffstrasse 3
D-5653 Leichlingen(DE)

(54) **Alkylen(cycloalkylen)-bis-heterocyclyl-biguanide.**

(57) Die vorliegende Erfindung betrifft neue Alkylen(cycloalkylen)-bis-heterocyclyl-biguanide der Formel (I)

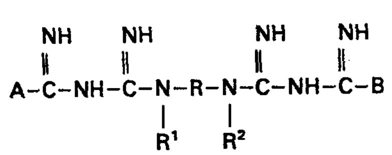

(I)

in welcher

A und B für einen über Stickstoff verknüpften heterocyclischen Rest,

R für gegebenenfalls alkylsubstituiertes Cycloalkylen oder Alkylen steht, wobei die Alkylenkette ein- oder mehrfach unterbrochen sein kann durch gegebenenfalls ein- oder mehrfach alkylsubstituierte Cycloalkylen-, Bicycloalkylen-, Tricycloalkylengruppen, durch Sauerstoffatome, Schwefelatome oder die Gruppe $NR^3$,

$R^1$ und $R^2$ für Wasserstoff, Alkyl oder gegebenenfalls substituiertes Aralkyl stehen oder gemeinsam mit dem Alkylenrest und den Stickstoffatomen einen heterocyclischen Ring bilden und

$R^3$ für Wasserstoff, Alkyl oder gegebenenfalls substituiertes Aralkyl steht.

Man erhält die Verbindungen durch Umsetzung von Alkylen-bis-dicyanamid mit Heterocyclen gegebenenfalls in Gegenwart eines Lösungsmittels.

Die neuen Alkylen(cycloalkylen)-bis-heterocyclylbiguanide der Formel (I) können als Schädlingsbekämpfungsmittel eingesetzt werden.

EP 0 096 280 A2

0096280

BAYER AKTIENGESELLSCHAFT 5090 Leverkusen, Bayerwerk

Zentralbereich
Patente, Marken und Lizenzen Bas/ABc
Ia

## Alkylen(cycloalkylen)-bis-heterocyclyl-biguanide

Die vorliegende Erfindung betrifft neue Alkylen(cycloalkylen)-bis-heterocyclyl-biguanide, ein Verfahren zu ihrer Herstellung und ihre Verwendung als Schädlingsbekämpfungsmittel.

Es ist bereits bekannt geworden, daß Alkylen-bis-arylbiguanide, wie z.B. das Chlorhexidin der Formel

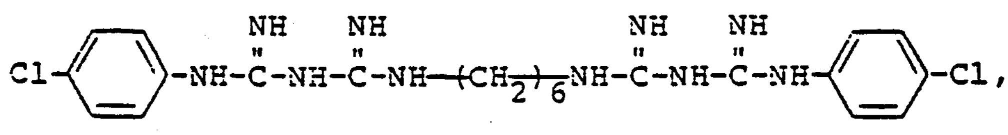

im technichen Bereich und im Humanbereich zu Desinfektionszwecken eingesetzt werden (vgl. Ullmann's Encyclopädie der technischen Chemie, 4. Auflage, Band 10, Seite 54, Verlag Chemie Weinheim 1975). Über eine Wirkung gegen Schädlinge im Pflanzenschutz ist nichts bekannt.

Weiterhin ist seit langem bekannt, daß N-sulfenylierte Dicarbonsäureimide, wie das N-Trichlormethylthio-tetrahydrophthalimid, eine fungizide Wirksamkeit besitzen (vgl. DBP 1 193 498).

Es wurden neue Alkylen(cycloalkylen)-bis-heterocyclyl-biguanide der Formel (I) gefunden

$$A-\overset{NH}{\overset{\|}{C}}-NH-\overset{NH}{\overset{\|}{C}}-\underset{R^1}{\underset{|}{N}}-R-\underset{R^2}{\underset{|}{N}}-\overset{NH}{\overset{\|}{C}}-NH-\overset{NH}{\overset{\|}{C}}-B \qquad (I),$$

in welcher

A und B gleich oder verschieden sind und für über Stickstoff verknüpfte heterocyclische Reste stehen, die gegebenenfalls substituiert sein können,

R für gegebenenfalls alkylsubstituiertes Cycloalkylen, oder Alkylen steht, wobei die Alkylenkette ein- oder mehrfach unterbrochen sein kann durch gegebenenfalls ein- oder mehrfach alkylsubstituierte Cycloalkylen-, Bicycloalkylen-, Tricycloalkylengruppen, durch Sauerstoffatome, Schwefelatome oder die Gruppe $NR^3$,

$R^1$ und $R^2$ gleich oder verschieden sind und für Wasserstoff, Alkyl oder gegebenenfalls substituiertes Aralkyl stehen oder

$R^1$ und $R^2$ unter Einbeziehung der beiden Stickstoffatome, an denen sie stehen, und des Alkylenrestes einen heterocyclischen Ring bilden und

$R^3$ für Wasserstoff, Alkyl oder gegebenenfalls substituiertes Aralkyl steht.

Weiterhin wurde gefunden, daß man die Alkylen-(cycloalkylen)-bis-heterocyclyl-biguanide der Formel (I)

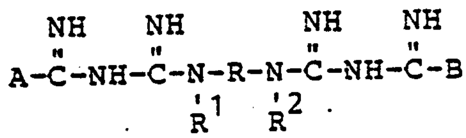

(I),

in welcher

A und B gleich oder verschieden sind und für über Stickstoff verknüpfte heterocyclische Reste stehen, die gegebenenfalls substituiert sein können,

R für gegebenenfalls alkylsubstituiertes Cycloalkylen, oder Alkylen steht, wobei die Alkylenkette ein- oder mehrfach unterbrochen sein kann durch gegebenenfalls ein- oder mehrfach alkylsubstituierte Cycloalkylen-, Bicycloalkylen-, Tricycloalkylengruppen oder durch Sauerstoffatome, Schwefelatome oder die Gruppe $NR^3$,

$R^1$ und $R^2$ gleich oder verschieden sind und für Wasserstoff, Alkyl oder gegebenenfalls substituiertes Arylalkyl stehen oder

$R^1$ und $R^2$ unter Einbeziehung der beiden Stickstoffatome, an denen sie stehen, und des Alkylenrestes einen heterocyclischen Ring bilden, und

$R^3$ für Wasserstoff, Alkyl oder gegebenenfalls substituiertes Arylalkyl steht, erhält, wenn man

a) für den Fall, daß A und B gleich sind, Alkylen-bis-dicyandiamide der Formel (II)

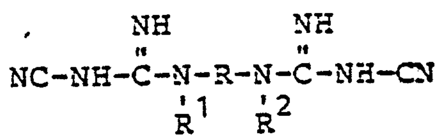 (II),

in welcher

R, $R^1$ und $R^2$ die oben angegebene Bedeutung haben, mit einem Heterocyclus der Formel

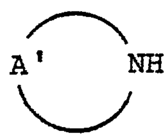 (III) bzw. 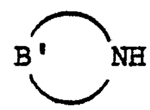 (IIIa)

in welchen

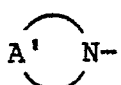 und 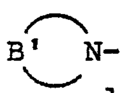 die oben angegebene Bedeutung von A und B haben, als freie Verbindungen oder in Salzform, gegebenenfalls in einem Lösungs- oder Verdünnungsmittel bei Temperaturen zwischen 50 und 200°C umsetzt, oder

b) für den Fall, daß A und B verschieden sind, Alkylen-bis-dicyandiamide der Formel (II) mit 1 Mol eines Heterocyclus der Formel (III) und anschließend mit 1 Mol des Heterocyclus der Formel (IIIa) gegebenenfalls in einem Lösungs- oder Verdünnungsmittels bei Temperaturen von 50 bis 200°C umsetzt.

Die neuen Alkylen(cycloalkylen)-bis-heterocyclyl-biguanide weisen starke fungizide Eigenschaften auf. Dabei zeigen die erfindungsgemäßen Verbindungen der Formel (I) überraschenderweise eine bessere fungizide Wirksamkeit als das aus dem Stand der Technik bekannte N-Trichlormethylthio-tetrahydrophthalimid. Die erfindungsgemäßen Stoffe stellen somit eine Bereicherung der Technik dar.

Die erfindungsgemäßen Alkylen(cycloalkylen)-bis-heterocyclyl-biguanide sind durch die Formel (I) allgemein definiert. In dieser Formel stehen vorzugsweise

A und B für gleiche oder verschiedene, über Stickstoff verknüpfte, gegebenenfalls durch weitere Heteroatome unterbrochene, gegebenenfalls ein- oder mehrfach,gleich oder verschieden substituierte 5- oder 6-gliedrige Ringe, die gegebenenfalls benzoannelliert sind. Als Substituenten kommen in Frage: Halogen, Nitro, Alkyl mit 1 bis 18 Kohlenstoffatomen, Alkylthio mit 1 bis 10 Kohlenstoffatomen und gegebenenfalls ein- bis sechsfach, gleich oder verschieden durch Halogen, Alkyl mit 1 bis 4 Kohlenstoffatomen und Halogenalkyl mit 1 bis 3 Kohlenstoffatomen und bis zu fünf Halogenatomen substituiertes Aryl mit 6 bis 10 Kohlenstoffatomen. Der ankondensierte Benzolring kann gegebenenfalls ein- bis vierfach durch die bei den Heterocyclen aufgezählten Substituenten substituiert sein.

R für Cycloalkyl mit 5 bis 9 Kohlenstoffatomen im Ring, der gegebenenfalls ein- oder mehrfach, gleich oder verschieden durch Alkyl mit 1 bis 6 Kohlenstoffatomen substituiert ist,

oder für geradkettiges oder verzweigtes Alkylen mit 1 bis 20 Kohlenstoffatomen, wobei die Alkylenkette gegebenenfalls ein- oder mehrfach substituiert ist durch Cycloalkylenreste mit 5 bis 7 Kohlenstoffatomen im Ring, durch Bicyclo- bzw. Tricycloalkylenreste mit 6 bis 18 Kohlenstoffatomen in den Ringen, wobei die Ringe ein- oder mehrfach durch Niederalkyl mit 1 bis 4 Kohlenstoffatomen substituiert sein können, durch Sauerstoff- oder Schwefelatome oder durch die Gruppe $NR^3$,

$R^1$ und $R^2$ gleich oder verschieden sind und für Wasserstoff, Alkyl mit 1 bis 20 Kohlenstoffatomen oder für Aralkyl mit 6 bis 10 Kohlenstoffatomen im Aryl- und 1 bis 6 Kohlenstoffatomen im Alkylteil steht, wobei der Arylteil ein- bis fünffach, gleich oder verschieden durch Alkyl mit 1 bis 6 Kohlenstoffatomen und Halogen substituiert sein kann oder

$R^1$ und $R^2$ unter Einbeziehung des Alkylenrestes und der Stickstoffatome, an denen die Reste $R^1$ und $R^2$ stehen, für einen heterocyclischen Ring mit 5 bis 10 Ringgliedern und

$R^3$ für Wasserstoff, Alkyl mit 1 bis 6 Kohlenstoffatomen oder für gegebenenfalls substituiertes Aralkyl mit 6 bis 10 Kohlenstoffatomen im Aryl- und 1 bis 6 Kohlenstoffatomen im Alkylteil, wobei der Arylteil ein- bis fünffach, gleich oder verschieden durch Alkyl mit 1 bis 6 Kohlenstoffatomen und Halogen substituiert sein kann.

Besonders bevorzugt sind Verbindungen der Formel (I), in denen

A und B gleich sind und für einen gegebenenfalls ein- bis dreifach, gleich oder verschieden durch Halogen, wie Chlor, Fluor und Brom, Nitro, Alkyl mit 1 bis 8 Kohlenstoffatomen, wie Methyl, Ethyl, n-Propyl, iso-Propyl, n-Butyl, iso-Butyl, tert.-Butyl, sec.-Butyl, Pentyl, Hexyl, Octyl und Nonyl, Alkylthio mit 1 bis 4 Kohlenstoffatomen, wie Methylthio, Ethylthio, n-Propylthio, iso-Propylthio, n-Butylthio, iso-Butylthio, sec.-Butylthio und tert.-Butylthio oder durch Phenyl substituierten Triazol-, wie 1,2,4-Triazol-, 1,3,4-Triazol- und 1,2,3-Triazol-, Tetrazol-, Pyrazol- oder Imidazolreste stehen, ferner für Benzotriazol- und Benzimidazolreste, wobei der ankondensierte Benzolring gegebenenfalls ein- bis vierfach, gleich oder verschieden durch Halogen, wie Fluor, Chlor und Brom, Nitro, Alkyl mit 1 bis 6 Kohlenstoffatomen, wie Methyl, Ethyl, n-Propyl, iso-Propyl, n-Butyl, iso-Butyl, sec.-Butyl, tert.-Butyl, Pentyl und Hexyl oder

Alkylthio mit 1 bis 4 Kohlenstoffatomen, wie Methylthio, Ethylthio, n-Propylthio, iso-Propylthio, n-Butylthio, sec.-Butylthio, iso-Butylthio und tert.-Butylthio, substituiert ist, der Phenylsubstituent kann gegebenenfalls substituiert sein durch Methyl, Ethyl, Propyl, Fluor, Chlor, Trifluormethyl oder Difluorchlormethyl,

R für Cycloalkylen mit 5 bis 7 Kohlenstoffatomen im Ring, wie Cyclopentylen, Cyclohexylen und Cycloheptylen, wobei die Ringe gegebenenfalls durch Alkyl mit 1 bis 4 Kohlenstoffatomen, wie Methyl, Ethyl, n-Propyl, iso-Propyl, n-Butyl, iso-Butyl, sec.-Butyl oder tert.-Butyl, ein- oder mehrfach substituiert sind,

für geradkettiges oder verzweigtes Alkylen mit 1 bis 12 Kohlenstoffatomen, wie Methylen, Ethylen, iso-Propylen, n-Propylen, n-Butylen, iso-Butylen, sec.-Butylen, Tetramethylen, Pentamethylen, Hexamethylen, Heptamethylen, Octamethylen, Nonamethylen, Decamethylen, 2,5-Dimethylhexylen-, 3,5,5-Trimethyl-hexylen, 1-Methylethylen, durch Cycloalkylen, Bi- oder Tricycloalkylen mit 5 bis 12 Kohlenstoffatomen im Ringsystem ein- oder mehrfach substituierte Alkylenreste mit 1 bis 4 Kohlenstoffatomen je Alkylenrest, wie 1,3,3-Trimethyl-cyclohexylmethylen, Dicyclohexylenmethan, 2,2'-Dimethyl-dicyclohexylenmethan, Bismethylentricyclodekan, für gegebenenfalls ein- oder mehrfach durch Sauerstoff, Schwefel oder $NR^3$ unterbrochene Alkylenreste mit 1 bis 6 Kohlenstoffatomen, wie N,N'-Bisethylen-ethylendiamin, Tetraethylentriamin, Pentaethylentetramin, Dipropylenamin, Tripropylenamin, 1,4-Butandiolbis-propylenether, stehen und

$R^1$ und $R^2$ gleich oder verschieden sind und für Wasserstoff, Alkyl mit 1 bis 10 Kohlenstoffatomen, wie Methyl, Ethyl, n-Propyl, iso-Propyl, n-Butyl, sec.-Butyl, iso-Butyl, tert.-Butyl, Pentyl, Hexyl, Heptyl, Octyl, Nonyl oder Decyl, oder für Aralkyl, wie Phenethyl oder Benzyl, steht, wobei der Arylrest ein- bis fünffach, gleich oder verschieden durch Alkyl mit 1 bis 4 Kohlenstoffatomen, wie Methyl, Ethyl, n-Propyl, iso-Propyl, n-Butyl, sec.-Butyl, iso-Butyl und tert.-Butyl, und Halogen, wie Fluor, Chlor, Brom und Jod, substituiert sein kann oder

$R^1$ und $R^2$ unter Einbeziehung des Alkylenrestes und der Stickstoffatome, an denen die Reste $R^1$ und $R^2$ stehen, einen heterocyclischen Ring mit 5 bis 10 Ringgliedern bilden und

$R^3$ für Wasserstoff, für Alkyl mit 1 bis 4 Kohlenstoffatomen, wie Methyl, Ethyl, n-Propyl, iso-Propyl, n-Butyl, sec.-Butyl, iso-Butyl und tert.-Butyl, oder für Aralkyl, wie Benzyl und Phenethyl, steht, wobei der Arylrest durch Alkyl mit 1 bis 4 Kohlenstoffatomen und/oder Fluor, Chlor, Brom, Jod substituiert sein kann.

Verwendet man beispielsweise bei der erfindungsgemäßen Verfahrensvariante a) Hexamethylen-bis-dicyan-diamid und 1,2,4-Triazol als Ausgangsverbindungen, so kann der

Reaktionsverlauf durch das folgende Formelschema wiedergegeben werden:

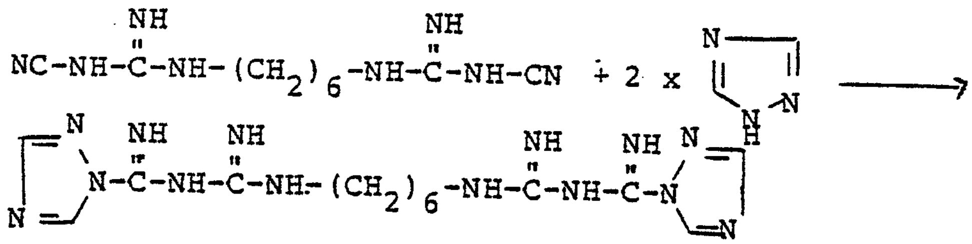

Verwendet man beispielsweise bei der erfindungsgemäßen Verfahrensvariante b) Tetramethylen-bis-dicyandiamid und 1,2,4-Triazol im ersten Schritt und Benzimidazol im zweiten Schritt als Ausgangsmaterialien, so kann der Reaktionsverlauf durch das folgende Formelschema wiedergegeben werden:

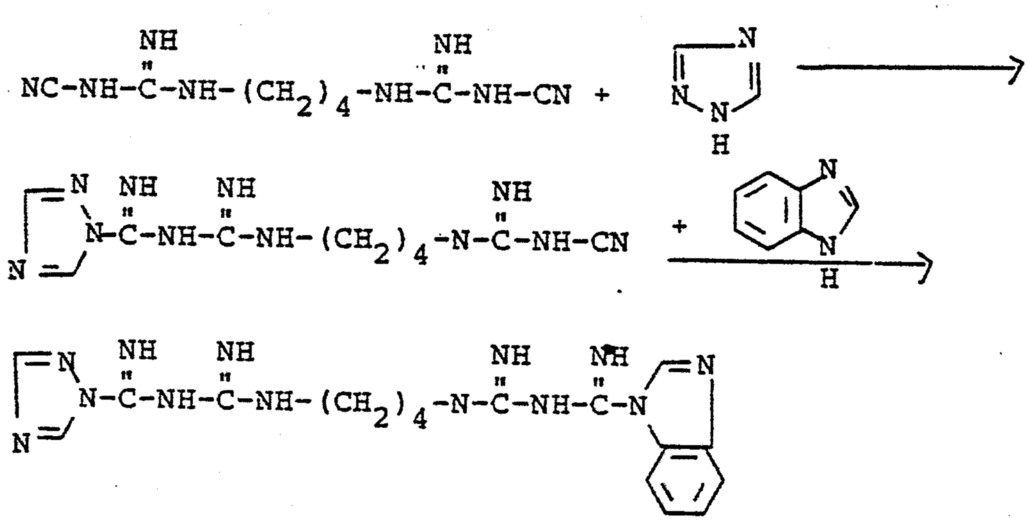

Die bei der Durchführung der erfindungsgemäßen Verfahrensvarianten a) und b) als Ausgangsstoffe zu verwendenden Heterocyclen der Formel III bzw. IIIa bzw. deren

Salze sind größtenteils bekannt oder nach bekannten Verfahren herstellbar.

Die außerdem bei der Variante a) als Ausgangsstoffe einzusetzenden Alkylen-bis-dicyandiamide der Formel (II) sind größtenteils bekannt oder nach bekannten Verfahren herstellbar (vgl. Britische Patente 631.878 und 702.268).

Für die erfindungsgemäße Umsetzung nach Verfahrensvariante a) und b) kommen als Verdünnungs- oder Lösungsmittel organische Lösungsmittel in Frage. Hierzu gehören vorzugsweise Alkohole, wie Methanol, Ethanol, iso-Propanol, n-Propanol, n-Butanol, iso-Butanol, Amylalkohol, Cyclohexanol, Phenol; Glykole und Glykolether, wie Ethylenglykol, Ethylenglykolmono- und dialkylether, Diethylenglykol; sowie Kohlenwasserstoffe und Halogenkohlenwasserstoffe, wie Toluol, Chloraromaten, Paraffinöl; Carbonsäuren, wie Ameisensäure, Essigsäure und Propionsäure, außerdem auch Wasser.

Bevorzugt wird ohne Lösungsmittel oberhalb des Schmelzpunktes des Reaktionsgemisches gearbeitet.

Die Reaktion kann bei Normal-, Unter- oder auch Überdruck durchgeführt werden. Bevorzugt wird bei Normaldruck gearbeitet.

Die Reaktionstemperaturen können bei der Durchführung des erfindungsgemäßen Verfahrens in einem größeren Bereich variiert werden. Im allgemeinen arbeitet man zwischen 50°C und 200°C, vorzugsweise zwischen 100°C und 170°C.

Der Verlauf der Reaktion ist durch Abnahme der CN-Bande im IR-Spektrum gut zu verfolgen, das Reaktionsende zeigt sich demnach durch Verschwinden der CN-Bande, es kann jedoch selbstverständlich auch früher abgebrochen werden, wo z.B. die thermische Stabilität der Einsatz- oder Endprodukte nicht ausreicht, um einen 100 %igen Umsatz ohne Ausbeuteverlust zu erzielen.

Die Aufarbeitung und Reinigung erfolgt durch übliche Methoden, wie z.B. Zerkleinern, Auflösen, Filtrieren, Eindampfen, Umkristallisieren.

In einer bevorzugten Ausführungsform werden die Heterocyclen in Salzform eingesetzt.

Es können sowohl die Salze organischer, wie auch anorganischer Säuren verwendet werden, wie z.B. der Ameisensäure, Essigsäure, Salzsäure, Schwefelsäure, Phosphorsäure. Bevorzugt werden die Hydrochloride eingesetzt. Dabei kann auch die Salzbildung in einem unmittelbar der Reaktion vorausgehenden Verfahrensschritt durchgeführt werden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

So können z.B. fungizide Mittel im Pflanzenschutz eingesetzt werden zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Im Pflanzenschutz können die erfindungsgemäßen Wirkstoffe mit besonders gutem Erfolg zur Bekämpfung von Botrytis Arten, gegen pilzliche Krankheitserreger am Getreide, wie z.B. gegen Erysiphe-, Septoria-, Puccinia- und Pyrenophora-Arten, außerdem gegen Pellicularia an Reis eingesetzt werden.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnapthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmiteln oder Trägerstoffen sind solche Flüssigkeiten gemeint, welche bei normaler Temperatur und unter Normaldruck geasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus anorganischem Material wie Sägemehl,

Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol und -Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,00001 bis 0,02 %, am Wirkungsort erforderlich.

## Herstellungsbeispiele:

### Beispiel 1

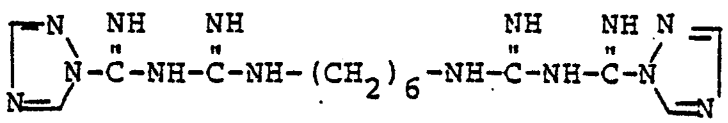

2 x HCl

25 g (0,1 Mol) Hexamethylen-bis-dicyandiamid, 21 g (0,2 Mol) 1,2,4-Triazol-hydrochlorid und 20 ml Wasser werden unter Abdestillieren des Wassers auf eine Innentemperatur von 145-150°C gebracht. Bei dieser Temperatur wird 5-10 Stunden langsam weitergerührt bis die CN-Bande im IR-Spektrum verschwunden ist. Dann wird die klare, zähe Schmelze ausgegossen, abgekühlt und die gebildete harzartige Masse zerkleinert. Ohne die verbliebenen Reste im Reaktionsgefäß wird eine Ausbeute von 42 g (91 % der Theorie) an Hexamethylen-bis-triazol-1-yl-biguanid-dihydrochlorid erhalten.

### Beispiel 2

Es wird wie in Beispiel 1 verfahren, jedoch anstelle von Triazol 21 g (0,2 Mol) Imidazol-hydrochlorid verwendet. Die Ausbeute beträgt 43 g (93 % der Theorie) an harzig-zähem, durchscheinenden Produkt. Nach Umkristallisieren aus Wasser entstehen schuppig-spröde Kristalle mit dem Schmelzpunkt 172-178°C an Hexamethylen-bis-imidazol-1-yl-biguanid-dihydrochlorid.

Beispiel 3

13 g (0,1 Mol) 2-Methyl-nitroimidazol und 10 g (0,1 Mol) 36 %ige Salzsäure werden auf 60°C erwärmt, wobei sich das Hydrochlorid des Azols bildet. 12,5 g (0,05 Mol) Hexamethylen-bis-dicyandiamid werden zugegeben und im schwachen Stickstoffstrom solange Wasser abdestilliert bis die Innentemperatur 145-150°C beträgt. Nach 5-10 Stunden bei dieser Temperatur ist die CN-Bande im IR-Spektrum verschwunden. Die Aufarbeitung erfolgt wie im Beispiel 1 beschrieben. Die Ausbeute beträgt 27 g (94 % der Theorie) an harzartigem, leicht hygroskopischem Hexamethylen-bis-2-methyl-nitroimidazol-1-yl-biguanid-dihydrochlorid.

Analog Beispiel 3 können die folgenden Verbindungen hergestellt werden:

| Beispiel Nr. | Struktur | Schmelzpunkt /°C/ |
|---|---|---|
| 4 | 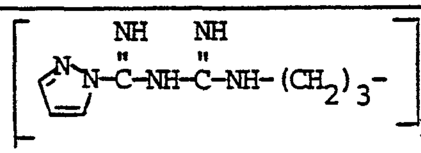 • 2HCl | 150-160 |
| 5 | 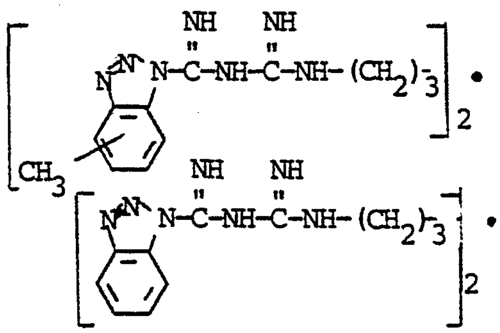 • 2HCl | 155-165 |
| 6 | • 2HCl | Harz |

| Beispiel Nr. | Struktur | Schmelzpunkt /°C/ |
|---|---|---|
| 7 | 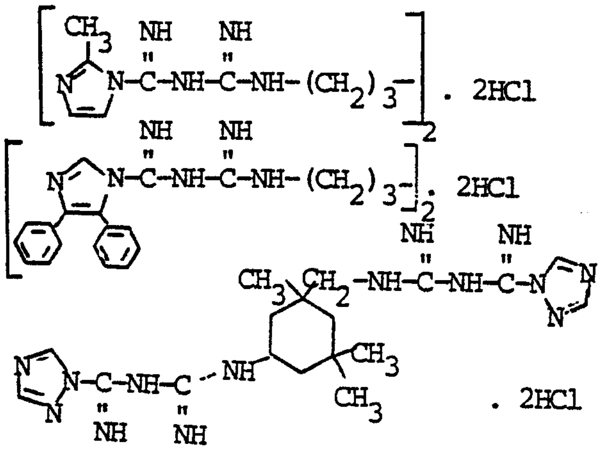 . 2HCl | Harz |
| 8 | . 2HCl | 187-192 |
| 9 | . 2HCl | 200 |
| 10 | 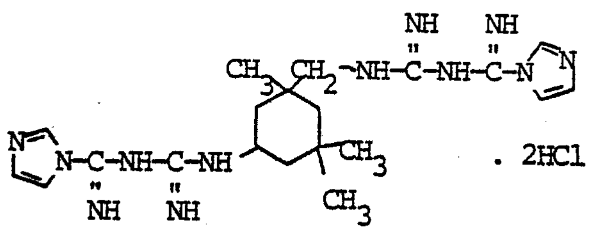 . 2HCl | Harz |
| 11 | 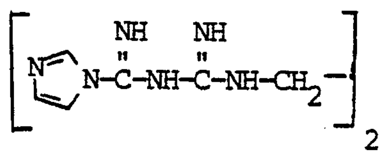 . 2HCl | Harz |
| 12 | 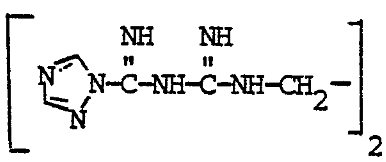 . 2HCl | Harz |

| Beispiel Nr. | Struktur | | Schmelzpunkt [°C] |
|---|---|---|---|
| 13 | 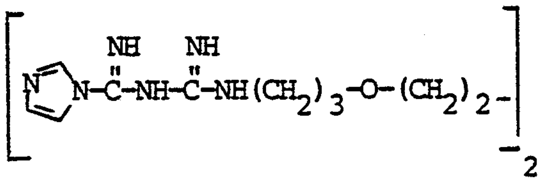 | . 2HCl | Harz |
| 14 | 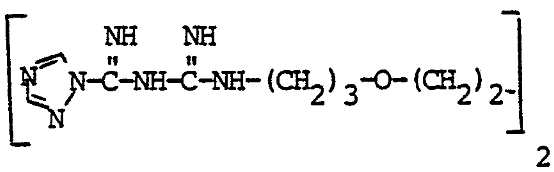 | . 2HCl | Harz |
| 15 | 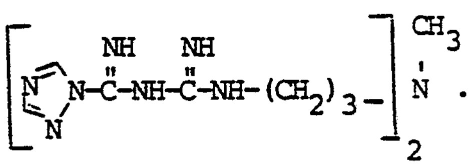 | . 3 HCl | Harz |
| 16 | 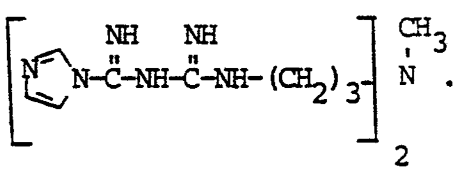 | . 3 HCl | Harz |
| 17 | 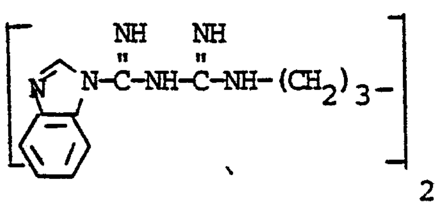 | . 2HCl | Harz |

Anwendungsbeispiele

In dem nachfolgenden Beispiel wird die nachstehend angegebene Verbindung als Vergleichssubstanz eingesetzt:

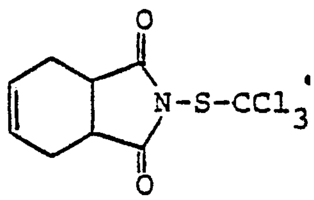

Beispiel

Botrytis-Test (Bohne) protektiv

Lösungsmittel: 4,7 Gewichtsteile Aceton
Emulgator: 0,3 Gewichtsteile Alkyl-aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in der Wirksamkeit gegenüber dem Stand der Technik zeigen in diesem Test z.B. die Verbindungen gemäß folgender Herstellungsbeispiele: 2, 1 und 3.

Patentansprüche

1. Alkylen(cycloalkylen)-bis-heterocyclyl-biguanide der Formel (I)

$$A-\overset{\overset{NH}{\|}}{C}-NH-\overset{\overset{NH}{\|}}{C}-\underset{\underset{R^1}{|}}{N}-R-\underset{\underset{R^2}{|}}{N}-\overset{\overset{NH}{\|}}{C}-NH-\overset{\overset{NH}{\|}}{C}-B \qquad (I),$$

in welcher

A und B gleich oder verschieden sind und für über Stickstoff verknüpfte heterocyclische Reste stehen, die gegebenenfalls substituiert sein können,

R für gegebenenfalls alkylsubstituiertes Cycloalkylen oder für gegebenenfalls durch Cycloalkylen, Bicycloalkylen, Tricycloalkylen, Sauerstoff, Schwefel oder die $NR^3$-Gruppe substituiertes Alkylen steht,

$R^1$ und $R^2$ gleich oder verschieden sind und für Wasserstoff, Alkyl oder gegebenenfalls substituiertes Aralkyl stehen oder

$R^1$ und $R^2$ unter Einbeziehung des Alkylenrestes und der beiden Stickstoffatome, an denen sie stehen, einen heterocyclischen Ring bilden und

$R^3$ für Wasserstoff, Alkyl oder gegebenenfalls substituiertes Aralkyl steht.

2. Alkylen(cycloalkylen)-bis-heterocyclyl-biguanide gemäß Anspruch 1, wobei in der Formel (I)

A und B für gleiche oder verschiedene, über Stickstoff verknüpfte, gegebenenfalls durch weitere Heteroatome unterbrochene, gegebenenfalls substituierte 5- oder 6-gliedrige Ringe, die gegebenenfalls benzoannelliert sind, stehen. Als Substituenten an den Ringen kommen in Frage: Halogen, Nitro, Alkyl mit 1 bis 18 Kohlenstoffatomen und Alkylthio mit 1 bis 10 Kohlenstoffatomen und gegebenenfalls ein- bis sechsfach, gleich oder verschieden durch Halogen, Alkyl mit 1 bis 4 Kohlenstoffatomen und Halogenalkyl mit 1 bis 3 Kohlenstoffatomen und bis zu 5 Halogenatomen substituiertes Aryl mit 6 bis 10 Kohlenstoffatomen,

R für gegebenenfalls durch Alkyl mit 1 bis 6 Kohlenstoffatomen substituiertes Cycloalkylen mit 5 bis 9 Ringkohlenstoffatomen, für gegebenenfalls durch Cycloalkylen mit 5 bis 7, Bicycloalkylen mit 6 bis 12, Tricycloalkylen mit 9 bis 18 Ringkohlenstoffatomen, Sauerstoff, Schwefel oder die $NR^3$-Gruppe substituiertes Alkylen mit 1 bis 20 Kohlenstoffatomen steht,

$R^1$ und $R^2$ gleich oder verschieden sind und für Wasserstoff, geradkettiges oder verzweigtes Alkyl mit 1 bis 20 Kohlenstoffatomen oder für gegebenenfalls durch Alkyl mit 1 bis 6 Kohlenstoffatomen und Halogen substituiertes Aralkyl mit 6 bis 10 Kohlenstoffatomen im Aryl- und 1 bis 6 Kohlenstoffatomen im Alkylteil stehen, oder

$R^1$ und $R^2$ unter Einbeziehung des Alkylenrestes und der Stickstoffatome, an denen die Reste $R^1$ und $R^2$ stehen, für einen heterocyclischen Ring mit 5 bis 10 Ringgliedern stehen und

$R^3$ für Wasserstoff, Alkyl mit 1 bis 6 Kohlenstoffatomen oder für gegebenenfalls durch Alkyl mit 1 bis 6 Kohlenstoffatomen und Halogen substituiertes Aralkyl mit 6 bis 10 Kohlenstoffatomen im Aryl- und 1 bis 6 Kohlenstoffatomen im Alkylteil steht.

3. Alkylen(cycloalkylen)-bis-heterocyclyl-biguanide gemäß Anspruch 1, wobei in der Formel (I)

A und B gleich sind und für gegebenenfalls durch Halogen, Nitro, Alkyl mit 1 bis 8 Kohlenstoffatomen oder Alkylthio mit 1 bis 4 Kohlenstoffatomen oder durch Phenyl substituiertes Triazol-, Tetrazol, Pyrazol oder Imidazol stehen, ferner für Benzotriazol- und Benzimidazolreste, wobei der

ankondensierte Benzolring gegebenenfalls durch Halogen, Nitro, Alkyl mit 1 bis 4 Kohlenstoffatomen oder Alkylthio mit 1 bis 4 Kohlenstoffatomen substituiert ist und der Phenylsubstituent gegebenenfalls durch Methyl, Ethyl, Propyl, Fluor, Chlor, Trifluormethyl oder Difluormethyl substituiert ist,

R für Cycloalkylen mit 5 bis 7 Kohlenstoffatomen im Ring steht, wobei die Ringe gegebenenfalls durch Alkyl mit 1 bis 4 Kohlenstoffatomen, ein- oder mehrfach substituiert sind, für geradkettiges oder verzweigtes Alkylen mit 1 bis 12 Kohlenstoffatomen, für durch Cycloalkylen, Bicyclo- bzw. Tricycloalkylenreste mit 5 bis 12 Kohlenstoffatomen im Ringsystem oder durch Sauerstoffatome, Schwefelatome oder die $NR^3$-Gruppe substituiertes Alkylen mit 1 bis 6 Kohlenstoffatomen je Alkylenrest und

$R^1$ und $R^2$ gleich oder verschieden sind und für Wasserstoff, Alkyl mit 1 bis 10 Kohlenstoffatomen, oder für Aralkyl stehen, wobei der Arylrest gegebenenfalls durch Alkyl mit 1 bis 4 Kohlenstoffatomen und Halogen, substituiert ist, oder

$R^1$ und $R^2$ unter Einbeziehung des Alkylenrestes und der Stickstoffatome, an denen die Reste $R^1$ und $R^2$ stehen, einen heterocyclischen Ring mit 5 bis 10 Ringgliedern bilden und

$R^3$ für Wasserstoff, für Alkyl mit 1 bis 4 Kohlenstoffatomen, oder für Aralkyl, steht, wobei der Arylrest gegebenenfalls durch Alkyl mit 1 bis 4 Kohlenstoffatomen und Fluor, Chlor, Brom oder Jod substituiert ist.

4. Verfahren zur Herstellung von Alkylen(cycloalkylen)-bis-heterocyclyl-biguaniden der Formel (I)

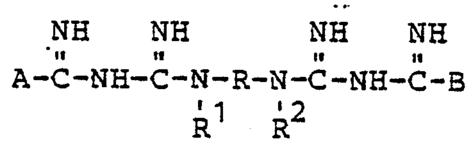

(I)

in welcher

A und B gleich oder verschieden sind und für über Stickstoff verknüpfte heterocyclische Reste stehen, die gegebenenfalls substituiert sind,

R für gegebenenfalls alkylsubstituiertes Cycloalkylen oder für gegebenenfalls durch Cycloalkylen, Bicycloalkylen, Tricycloalkylen, Sauerstoff, Schwefel oder die $NR^3$-Gruppe substituiertes Alkylen steht,

$R^1$ und $R^2$ gleich oder verschieden sind und für Wasserstoff, Alkyl oder gegebenenfalls substituiertes Aralkyl stehen oder

$R^1$ und $R^2$ unter Einbeziehung der beiden Stickstoffatome, an denen sie stehen, und des Alkylenrestes einen heterocyclischen Ring bilden, und

$R^3$ für Wasserstoff, Alkyl oder gegebenenfalls substituiertes Aralkyl steht, da-

a) für den Fall, daß A und B gleich sind, Alkylen-bis-dicyanamide der Formel (II)

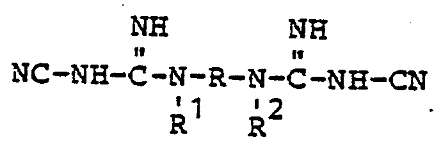

(II),

in welcher

R, $R^1$ und $R^2$ die oben angegebene Bedeutung haben, mit einem Heterocyclus der Formel

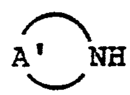 (III) bzw. 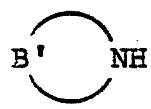 (IIIa),

in welchen

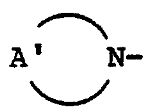 und 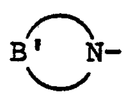 die oben angegebene Bedeutung von A und B haben, als freie Verbindungen oder in Salzform, gegebenenfalls in einem Lösungs- oder Verdünnungsmittel bei Temperaturen zwischen 50 und 200°C umsetzt, oder

b) für den Fall, daß A und B verschieden sind, Alkylen-bis-dicyanamide der Formel (II) mit 1 Mol eines Heterocyclus der Formel (III) und anschließend mit 1 Mol des Heterocyclus der Formel (IIIa) gegebenenfalls in einem Verdünnungsmittels bei Temperaturen von 50 bis 200°C umsetzt.

5. Schädlingsbekämpfungsmittel, gekennzeichnet durch einen Gehalt an mindestens einem Alkylen(cycloalkylen)-bis-heterocyclyl-biguanid der Formel (I), gemäß Ansprüchen 1 und 4.

6. Verwendung von Alkylen(cycloalkylen)-bis-heterocyclyl-biguaniden der Formel (I), gemäß Ansprüchen 1 und 4, zur Bekämpfung von Schädlingen.

7. Verfahren zur Bekämpfung von Schädlingen, dadurch gekennzeichnet, daß man Alkylen(cycloalkylen)-bis-heterocyclyl-biguanide der Formel (I),gemäß Ansprüchen 1 und 4, auf Schädlinge und/oder ihren Lebensraum einwirken läßt.

8. Verfahren zur Herstellung von Schädlingsbekämpfungsmitteln, dadurch gekennzeichnet, daß man Alkylen-(cycloalkylen)-bis-heterocyclyl-biguanide der Formel (I), gemäß Ansprüchen 1 und 4, mit Streckmitteln und/oder oberflächenaktiven Mitteln vermischt.